# EUROPÄISCHE PATENTSCHRIFT

(11) **EP 1 638 683 B1**
(45) Veröffentlichungstag und Bekanntmachung des Hinweises auf die Patenterteilung: **22.09.2010**
(21) Anmeldenummer: 04738563.8
(22) Anmeldetag: 26.05.2004
(51) Int. Cl.: B01J 20/30, A61F 13/15, D21H 23/44

(54) **VERFAHREN ZUM HERSTELLEN EINES ABSORBERHALTIGEN FASERPRODUKTES**
METHOD FOR PRODUCING AN ABSORBER-CONTAINING FIBROUS PRODUCT
PROCEDE DE PRODUCTION D'UN PRODUIT FIBREUX CONTENANT UN ABSORBEUR

(30) Priorität: 24.06.2003 DE 10328567
(43) Veröffentlichungstag der Anmeldung: 29.03.2006
(73) Patentinhaber: SAUERESSIG GMBH & CO., 48691 Vreden (DE)
(72) Erfinder: SAUERESSIG, Kilian, 48691 Vreden (DE)
(74) Vertreter: Tönhardt, Marion
(86) Internationale Anmeldenummer: PCT/DE2004/001094
(87) Internationale Veröffentlichungsnummer: WO 2005/007285

(56) Entgegenhaltungen:
- EP-A- 1 110 521
- WO-A-03/059228
- FR-A- 2 759 388
- US-A- 4 882 204
- US-A- 5 248 524
- US-A1- 2001 021 453

## Beschreibung

Die Erfindung betrifft ein Verfahren zum Herstellen eines absorberhaltigen Faserproduktes.

Bei heute bekannten Faserprodukten, die mit einem Absorber, beispielsweise quer vernetztem Natriumpolyacrylat, versehen sind, ist dieser in der Regel in Form von Pulver zwischen zwei oder mehreren Lagen des Faserproduktes eingestreut. Bei solchen Faserprodukten besteht die Gefahr, daß der Absorber ausrieselt und die Absorptionsfähigkeit verloren geht. Deshalb werden als alternative Vorgehensweise die Fasern mit einem Absorber beschichtet, bevor sie anschließend zu einem Faserprodukt verarbeitet werden.

In US-A-4 882 204 wird ein Spray für Windeln offenbart, wobei das Spray ein Aerosol mit Pulver eines Absorbers umfaßt. Das Aerosol weist dabei neben Alkohol und Wasser ein Treibgas auf. Als Treibgase kommen beispielsweise FREONe, CO₂, N₂ oder CH₂Cl₂ in Frage. Mittels des Treibgases wird das Aerosol aus einer Spraydose freigesetzt.

Das zusätzlich zu Alkohol und Wasser zum Freisetzen des Absorbers erforderliche Treibgas begründet bezüglich zusätzlicher Kosten und der Umweltproblematik einen Nachteil von US-A-4 882 204.

EP-A-1 110 521 beschreibt die Applikation eines Superabsorbers mit Hilfe einer Walze für die Herstellung von Windeln, Binden oder ähnlichen Faserprodukten. Um die Teilchen des Absorbers im porösen Netzwerk der Faserprodukte zu streuen, werden sie mit Luft als Trägermedium mittels eines Sauggebläses durch eine Rohrleitung auf das Faserprodukt auf einer Walze angesaugt. Die Saugkraft wird dabei im verschiedenen Ausmaß von vier verschiedenen Kammern der Walze ausgeübt, von denen für jede Kammer der statische Druck oder die Ansaugmenge an Luft separat eingestellt werden kann.

Das in EP-A-1 110 521 dargelegte Verfahren erfordert aufgrund der verschiedenen Kammern eine komplex konstruierte Walze. Darüber hinaus muß die Teilchenzuführungsöffnung so justiert werden, daß eine gleichmäßige Verteilung des Absorbers im Faserprodukt gewährleistet wird.

Zusätzlich müssen die verschiedenen Saugkräfte der einzelnen Kammern ebenfalls jeweils genau eingestellt werden. Die gleichzeitige Justierung von mehreren Parametern gestaltet sich meist als sehr aufwendig und von langer Dauer, da eine geeignete Kombination an Einstellung gefunden werden muß.

FR-A-2 759 388 offenbart ein Verfahren, bei dem Teilchen eines Absorbers aus einer zum Beispiel wäßrigen Dispersion heraus in das Faserprodukt eingebracht werden. Zum Binden der Absorberteilchen auf die Fasern im Innern des Faserprodukts werden Bindemittel eingesetzt, welche sich chemisch durch deren Bi-Funktionalität sowohl an den zu applizierenden Superabsorber einerseits, als auch an die Fasern des Faserprodukts andrerseits binden.

FR-A-2 759 388 weist den Nachteil auf, daß zum Applizieren der Absorber-Partikel aus einer wäßrigen Dispersion heraus zusätzlich ein Bindemittel erforderlich ist. Neben der schlechteren Wirtschaftlichkeit kann dieses Verfahren keine so homogene Verteilung, wie sie mit rein physikalischen Verfahren erzielbar wäre, gewährleisten. Da in diesem Verfahren vom Bindemittel mit den Absorber-Partikeln einerseits und mit den jeweiligen Fasern des Faserprodukts andererseits geschlossene chemische Verbindungen nicht wieder gelöst werden können, liegt nach dem Applizieren des Absorbers ein Verteilungsgradient von außen nach innen des Faserprodukts vor.

Das Anlagern erfolgt durch Wärmeeinwirkung, die es erlaubt, das Lösungsmittel verdampfen zu lassen. US-A-5 248 524 beschreibt das Einsprühen eines Superabsorbers mittels eines Gases, wie zum Beispiel Luft, oder aber, daß der Absorber selbst in einem fluiden Aggregatzustand versprüht wird. Zum Versprühen wird ein Düsen-Paar verwendet, von dem jede der beiden Düsen bezüglich des Abscheidungsprofils konfiguriert werden kann.

US-A-4 248 524 hat den Nachteil, daß die zwei Düsen des Düsenpaars genau justiert werden müssen, um ein geeignetes Abscheidungsprofil zu erhalten. Zusätzlich ist die Auswahl der einsetzbaren Absorber im Falle des Versprühens in deren fluidem Aggregatzustand signifikant eingeschränkt.

US 2001/0021453 A1 beschreibt ein Verfahren, bei dem ein Bindemittel erst aktiviert wird, wenn es zusammen mit dem Absorber in das Faserprodukt eingebracht wurde. Es ist die Aufgabe der vorliegenden Erfindung, ein Verfahren zum Herstellen eines absorberhaltigen Faserproduktes zur Verfügung zu stellen, mit dem die variable und selektive Einbringung von Absorbern unter Erhöhung der Saugfähigkeit und Naßreißfestigkeit des Faserproduktes gelingt.

Diese Aufgabe wird durch ein Verfahren nach Anspruch 1 gelöst. Vorteilhafte Ausgestaltungen sind Gegenstand der Unteransprüche.

Ein Dampf oder eine Emulsion wird erzeugt, der bzw. die ein Trägermedium und darin eingebettete Absorber als feinste Partikel enthält, wobei das Trägermedium eines ist, das flüssig ist und das mit dem Absorber nicht chemisch reagiert. Der Dampf oder die Emulsion wird dann in das Faserprodukt eingedüst. Erfindungsgemäβ ist die Temperatur des Faserproduktes derart, daß der Dampf oder die Emulsion an den Faseroberflächen kondensiert.

Wenn nach dem Eindüsen das Trägermedium verdampft oder verdunstet, geht der Absorber mit der Feuchtigkeit auf der Faser eine Verbindung ein. In der Regel wird die Feuchtigkeit Wasser sein, mit dem der Absorber eine irreversible Flüssigkeitsbindung eingeht. Der Absorber ist also dauerhaft im Faserprodukt gebunden und kann nicht ausrieseln. Bei herkömmlichen Produkten befindet sich der Absorber in hermetisch abgedichteten Taschen und ist nicht gleichmäßig im Produkt verteilt. Ebenso kann nach dem Stand der Technik der Absorber nur bei mehrlagigen Produkten eingesetzt werden, nicht bei einlagigen. Mit der Erfindung wird es möglich, Produkte mit geringen Materialstärken und niedrigen Flächengewichten im Bereich von 20 bis 30 g/m² hochsaugfähig zu gestalten, beispielsweise Küchenrollen, Taschentücher und dergleichen.

"Feinste Partikel" bedeutet dabei, daß die Partikelgröße des Absorbers so gewählt ist, daß die Partikel in das Innere des Faserproduktes vordringen können.

Die Wahl des Absorbers wird danach getroffen, welche Flüssigkeit absorbiert werden soll. In der Regel wird dies Wasser sein, so daß die bereits auf dem Markt befindlichen "Superabsorber" zum Einsatz kommen können. Gegebenenfalls müssen diese auf eine bestimmte Partikelgröße vermahlen werden, wie dies weiter unten erläutert ist.

Mit dem erfindungsgemäßen Verfahren werden die Fasern somit gezielt beschichtet, ohne daß sie komplett durchweicht würden. Es wird eine erhöhte Flüssigkeitsaufnahme vorzugsweise für wässerige Medien erzielt. Wenn die beschichtete Faser mit einer Flüssigkeit in Berührung kommt, so nimmt zunächst der Absorber die Flüssigkeit auf, erst später wird die Faser die Flüssigkeit aufnehmen. Außerdem kann mehr Flüssigkeit aufgenommen werden als mit einem Faserprodukt, das die erfindungsgemäße Beschichtung nicht zeigt. Die Fasern und die Berührungspunkte der Fasern werden durch den Absorber geschützt, was die Naßreißfestigkeit deutlich erhöht. Bei gleicher Flüssigkeitsaufnahme liegt sie gegenüber anderen Faserprodukten, die nicht beschichtet sind, deutlich höher. Bei der Quellung der Absorber tritt zusätzlich der Effekt ein, daß sich nah beieinander liegende Fasern voneinander entfernen. Damit wird für die zu absorbierende Flüssigkeit mehr Raum geschaffen, so daß die Flüssigkeitsaufnahme weiter erhöht wird.

Nach einer bevorzugten Ausgestaltung des Verfahrens wird das Faserprodukt vor oder nach dem Beschichten mit einer dosierten Menge einer Flüssigkeit, die chemisch mit dem Absorber reagiert, durchdampft. Dies bedeutet eine Vorexpansion bzw. Vorquellung des Absorbers, die für eine gute Lagenhaftung bzw. Faserhaftung im Faserprodukt sorgt. Wenn das Durchdampfen nach der Beschichtung erfolgt, werden nicht nur die Fasern, sondern insbesondere auch die Knotenpunkte vorgequollen, was das Volumen des Faserproduktes erhöht und dieses auch stabilisiert. Das Faserprodukt kann auch durchdampft werden, bevor die Eintragung des Absorbers erfolgt. Der Absorber reagiert dann mit der Feuchtigkeit, die sich an der Oberfläche der Fasern befindet. Auch hier werden die Fasern stabilisiert, insbesondere wird für einen besseren Zusammenhalt der Fasern in den Knotenpunkten gesorgt. Vorteilhaft ist, daß die Oberfläche des Absorbers, die von den Fasern weg gerichtet ist, nicht vorgequollen ist.

Der vorgequollene Absorber bewirkt auch eine Stabilisierung von Prägestrukturen, die zuvor in das Faserprodukt eingebracht worden sind. Die Prägestruktur bleibt bei den normalen Verarbeitungsprozessen und den dabei auftretenden Drücken erhalten und wird auch in einem abschließend stattfindenden Wickelprozeß nicht zerstört.

Bevorzugt ist die Korngröße der Absorberartikel kleiner als der mittlere Faserabstand, so daß der Absorber in das Faserbündel transportiert und nicht nur auf der Oberfläche des Faserproduktes abgeschieden wird. Daher ist es mit der Erfindung möglich, daß mehrlagige Faserprodukte auch nachträglich mit Absorbern versehen werden können. Es können folglich bestehende Anlagen nachgerüstet werden oder Neuanlagen können mit nur einer Beschichtungseinheit auskommen, auch wenn mehrere Lagen eines Faserproduktes verarbeitet werden. Das Einbringen der Absorber gelingt durch Walzen mit entsprechenden Öffnungen oder geeignet gewählte Düsenformen.

Je kleiner die Partikelgröße der Absorber ist, desto einfacher können sie in das Innere des Faserproduktes vordringen. Je nach gewähltem Absorber kann die Partikelgröße um ein Vielfaches kleiner sein als der mittlere Faserabstand. Der Absorber wird selektiv an den Fasern, nicht aber an den Freiräumen zwischen den Fasern abgeschieden, da das Aerosol oder der Dampf oder die Emulsion nur an den Fasern kondensiert. Es entsteht ein Produkt, bei dem der Absorber sehr gleichmäßig verteilt ist. Somit steht auch bei einem sehr kleinen Anteil an Absorber in dem Faserprodukt eine große Oberfläche im Verhältnis zum Absorbervolumen zur Verfügung. Die Absorptionsgeschwindigkeit von erfindungsgemäß hergestellten Faserprodukten ist daher deutlich höher als bei denen des Standes der Technik.

Das Trägermedium ist eine Flüssigkeit, insbesondere sind Alkohole oder Alkoholgemische bevorzugt, beispielsweise Methylalkohol oder Isopropanol.

Die in der vorstehenden Beschreibung und in den Ansprüchen offenbarten Merkmale der Erfindung können sowohl einzeln als auch in beliebiger Kombination für die Verwirklichung der Erfindung wesentlich sein.

## Patentansprüche

1. Verfahren zum Herstellen eines absorberhaltigen Faserproduktes, wobei ein Dampf oder eine Emulsion eines flüssigen Trägermediums, in welchem bzw. welcher feinste Partikel eines zum Trägermedium chemisch inerten Absorbers eingebettet sind, in ein Faserprodukt eingedüst wird,
**dadurch gekennzeichnet, dass** die Temperatur des Faserproduktes so gehalten wird, dass das Trägermedium des Dampfes oder der Emulsion an den Faseroberflächen kondensiert, sich die Partikel auf den Fasern ablagern und das Trägermedium das Faserprodukt wieder verlässt.

2. Verfahren nach Anspruch 1, **dadurch gekennzeichnet, dass** das Faserprodukt vor oder nach dem Eindüsen des Dampfes oder der Emulsion mit einer dosierten Menge einer Flüssigkeit, die chemisch mit dem Absorber reagiert, durchdampft wird, um mittels Flüssigkeitsbindung das Faserprodukt definiert vorquellen bzw. vorexpandieren zu lassen.

3. Verfahren nach Anspruch 1 oder 2, **dadurch gekennzeichnet, dass** die Korngröße der Absorberpartikel kleiner als der mittlere Faserabstand ist.

4. Verfahren nach Anspruch 1 oder 2, **dadurch gekennzeichnet, dass** das Trägermedium ein Alkohol oder ein Alkoholgemisch ist.

5. Verfahren nach Anspruch 1 oder 2, **dadurch gekennzeichnet, dass** das Trägermedium Methanol oder Isopropanol ist.

## Claims

1. A method for producing an absorber-containing fibrous product, wherein a vapour or an emulsion of a liquid carrier medium into which finest particles of an absorber chemically inert with respect to said carrier medium are embedded, is injected into a fibrous product,
**characterized in that** the temperature of said fibrous product is maintained such that the carrier medium of said vapour or said emulsion condenses at the fibre surfaces, said particles precipitate on the fibres and said carrier medium re-exits the fibrous product.

2. The method of claim 1, **characterized in that** the fibrous product is steamed by a dosed amount of a liquid which reacts chemically with said absorber, before or after injecting said vapour or said emulsion, in order to make the fibrous product pre-swell or pre-expand, respectively, by liquid bonding.

3. The method of claim 1 or 2, **characterized in that** the grain size of the absorber particles is smaller than the average fibre distance.

4. The method of claim 1 or 2, **characterized in that** said carrier medium is an alcohol or an alcohol mixture.

5. The method of claim 1 or 2, **characterized in that** said carrier medium is methanol or isopropanol.

## Revendications

1. Procédé pour fabriquer un produit fibreux contenant un absorbant, dans lequel une vapeur ou une émulsion d'un milieu porteur liquide est injectée dans un produit fibreux, vapeur ou émulsion dans laquelle sont incorporées les plus fines particules d'un absorbant chimiquement inerte par rapport au milieu porteur,
**caractérisé en ce que** la température du produit fibreux est maintenue de telle sorte que le milieu porteur de la vapeur ou de l'émulsion se condense sur les surfaces des fibres, les particules se déposent sur les fibres et le milieu porteur sort de nouveau du produit fibreux.

2. Procédé selon la revendication 1, **caractérisé en ce que** le produit fibreux est traité avant ou après l'injection de la vapeur ou de l'émulsion à l'aide d'une quantité dosée d'un liquide qui réagit chimiquement avec l'absorbant, pour permettre un gonflement préalable ou une expansion préalable de manière définie du produit fibreux au moyen d'une liaison liquide.

3. Procédé selon la revendication 1 ou 2, **caractérisé en ce que** la grosseur de grain des particules d'absorbant est inférieure à l'espacement moyen des fibres.

4. Procédé selon la revendication 1 ou 2, **caractérisé en ce que** le milieu porteur est un alcool ou un mélange d'alcools.

5. Procédé selon la revendication 1 ou 2, **caractérisé en ce que** le milieu porteur est le méthanol ou l'isopropanol.
